(19) Europäisches Patentamt
European Patent Office
Office européen des brevets

(11) **EP 3 378 560 A1**

(12) **EUROPEAN PATENT APPLICATION**

(43) Date of publication:
**26.09.2018 Bulletin 2018/39**

(51) Int Cl.:
**B01L 3/02** (2006.01)          **G01N 35/10** (2006.01)
**B41J 2/14** (2006.01)          **B41J 2/165** (2006.01)

(21) Application number: **18159392.2**

(22) Date of filing: **01.03.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.03.2017 JP 2017059795**

(71) Applicant: **Toshiba TEC Kabushiki Kaisha
Tokyo 141-8562 (JP)**

(72) Inventors:
• **HIGUCHI, Masaaki**
  **Shinagawa-ku,, Tokyo 141-8562 (JP)**
• **SHIMIZU, Seiya**
  **Shinagawa-ku,, Tokyo 141-8562 (JP)**
• **KUSUNOKI, Ryutaro**
  **Shinagawa-ku,, Tokyo 141-8562 (JP)**
• **YOKOYAMA, Shuhei**
  **Shinagawa-ku,, Tokyo 141-8562 (JP)**

(74) Representative: **Takeuchi, Maya et al
Fédit-Loriot
38, avenue Hoche
75008 Paris (FR)**

(54) **DROPLET DISPENSING APPARATUS**

(57)    According to one embodiment, a droplet dispensing apparatus includes a droplet ejection array having a plurality of nozzles from which droplets can be ejected into a well opening of a microplate plate on a baseplate, the plurality of nozzles being arranged in columns in a first direction and rows in a second direction that intersects the first direction, a light emitting unit configured to emit light having polarization perpendicular to a third direction oblique with respect to the first direction and the second direction, and a light receiving unit configured to receive light from the light emitting unit, the light receiving unit being on an opposite side of the droplet ejection array from the light emitting unit.

*FIG. 8*

## Description

FIELD

Embodiments described herein relate generally to the field of microanalysis, and more particularly to a droplet dispensing apparatus and an analytic system comprising the same.

BACKGROUND

In biological and pharmaceutical research and development, medical diagnosis and examination, and agricultural testing, liquid dispensing in a range of picoliter (pL) to microliter ($\mu$L) is often used.

For improved speed, a droplet ejecting apparatus typically ejects several droplets of liquid simultaneously from multiple nozzles into wells of a microwell plate or the like.

When liquid from a plurality of nozzles is being dispensed simultaneously, there is a possibility that some of the nozzles might not discharge the liquid as intended. In such a case, the intended fixed amount of liquid cannot be dispensed, which may cause erroneous evaluations in some testing applications.

SUMMARY OF INVENTION

To solve the above-cited problems, there is provided a droplet dispensing apparatus, comprising:

a droplet ejection array having a plurality of nozzles from which droplets can be ejected into a well opening of a microplate plate on a baseplate, the plurality of nozzles being arranged in columns in a first direction and rows in a second direction that intersects the first direction;
a light emitting unit configured to emit light having polarization perpendicular to a third direction oblique with respect to the first direction and the second direction; and
a light receiving unit configured to receive light from the light emitting unit, the light receiving unit being on an opposite side of the droplet ejection array from the light emitting unit.

In an exemplary embodiment preferably an inclination angle of the third direction with respect to the first direction is an acute angle.

Preferably in the embodiments when the number of columns is a, the number of rows is b, a distance between adjacent columns is X, a distance between adjacent rows is Y, and the inclination angle is $\theta$, a distance between a first nozzle, disposed in a first column and a first row, and an intersection point between a line through the first nozzle along the first direction and a line through a second nozzle, disposed in a second column and the first row, along the third direction is Z2, the second column being adjacent to the first column, the following relationships:

$$Z2 > Y \cdot (b\text{-}1),$$

and

$$0 < \tan\theta < X/Y \cdot (b\text{-}1)$$

are satisfied.

Preferably in the embodiments an inclination angle of the third direction with respect to the second direction is an acute angle.

The droplet dispensing apparatus according to the embodiments preferably may further comprise:
an additional droplet ejection unit configured to dispense liquid towards the microplate on the baseplate.

The droplet dispensing apparatus according to the embodiments preferably may further comprise:

a sealed box enclosing the droplet ejection array and at least a portion of the baseplate; and
a spraying device and configured to spray a liquid into the sealed box.

The droplet dispensing apparatus according to the embodiments preferably may further comprise:
a needle-like ejection member on the droplet ejection array configured to engage and open a lid on a periphery of a well opening of the microplate.

The droplet dispensing apparatus according to the embodiments preferably may further comprise:

a board having a first surface and a second surface, wherein the droplet ejection array is on the first surface of the board and the droplets can be ejected via the second surface of the board;
a plurality of pressure chambers on the second surface of the board, each pressure chamber in the plurality being fluidly connected to a respective nozzle in the plurality of nozzles;
a plurality of actuators that changes a pressure in a respective pressure chamber and causes liquid to be discharged from the respective nozzle; and
a plurality of solution holding containers on the second surface of the board, each having a solution receiving port for receiving liquid and a solution outlet for supplying liquid to a respective pressure chamber.

Preferably in the embodiments the light emitting unit and the light receiving unit are formed integrally with the droplet ejecting array.

In another exemplary embodiment there is also provided an analytic system comprising the droplet dispensing apparatus according to the embodiments.

DESCRIPTION OF THE DRAWINGS

The above and other objects, features and advantages of the present invention will be made apparent from the following description of the preferred embodiments, given as non-limiting examples, with reference to the accompanying drawings, in which:

FIG. 1 is a perspective view of a droplet dispensing apparatus according to a first embodiment.
FIG. 2 is a plan view of an upper surface a droplet ejecting apparatus.
FIG. 3 is a plan view of a lower surface of a droplet ejecting apparatus.
FIG. 4 is a cross-sectional view along line F4-F4 of FIG. 2.
FIG. 5 is a plan view of a droplet ejection array of a droplet ejecting apparatus.
FIG. 6 is a cross-sectional view along line F6-F6 of FIG. 5.
FIG. 7 is a schematic view of a droplet detection unit of a droplet dispensing apparatus.
FIG. 8 is a diagram of a droplet detection unit of a droplet dispensing apparatus.
FIG. 9 is depicts a minimum angle of an inclination angle of an optical path of a droplet detection unit.
FIG. 10 depicts a maximum angle of an inclination angle of an optical path of a droplet detection unit.
FIG. 11 is a perspective view of a droplet detection unit of a droplet dispensing apparatus according to a second embodiment.
FIG. 12 is a perspective view of a droplet detection unit of a droplet dispensing apparatus according to a third embodiment.
FIG. 13 is a perspective view of a droplet detection unit of a droplet dispensing apparatus according to a fourth embodiment.
FIG. 14 is a longitudinal cross-sectional view of a microplate according to a fifth embodiment.

DETAILED DESCRIPTION

In general, according to one embodiment, a droplet dispensing apparatus includes a droplet ejection array having a plurality of nozzles from which droplets can be ejected into a well opening of a microplate plate on a baseplate, the plurality of nozzles being arranged in columns in a first direction and rows in a second direction that intersects the first direction, a light emitting unit configured to emit light having polarization perpendicular to a third direction oblique with respect to the first direction and the second direction, and a light receiving unit configured to receive light from the light emitting unit, the light receiving unit being on an opposite side of the droplet ejection array from the light emitting unit.
Hereinafter, droplet dispensing apparatuses according to example embodiments will be described below with reference to the drawings. It should be noted, that the particular embodiments explained below are some possible examples of a droplet dispensing apparatus according to the present disclosure and do not limit the possible configurations, specifications, or the like of droplet dispensing apparatuses according to the present disclosure.

First embodiment

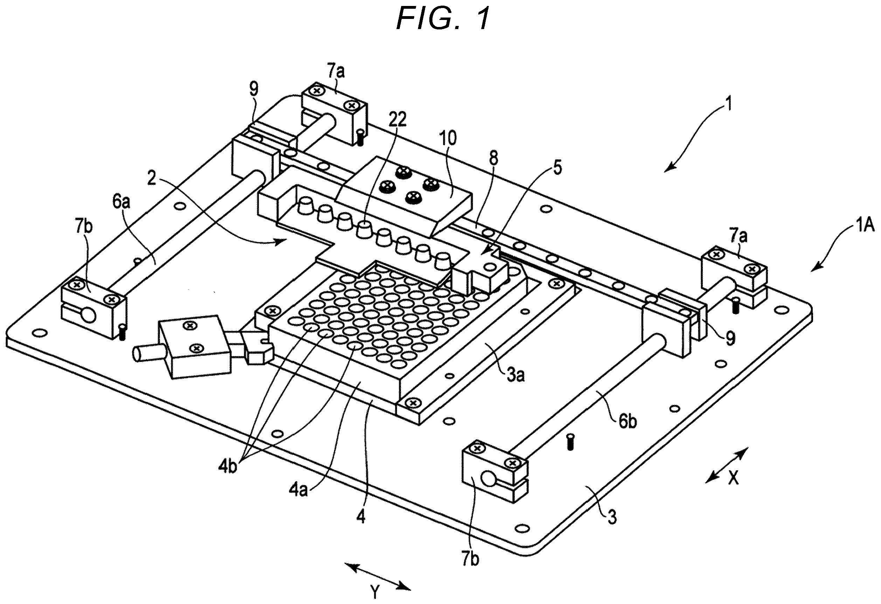

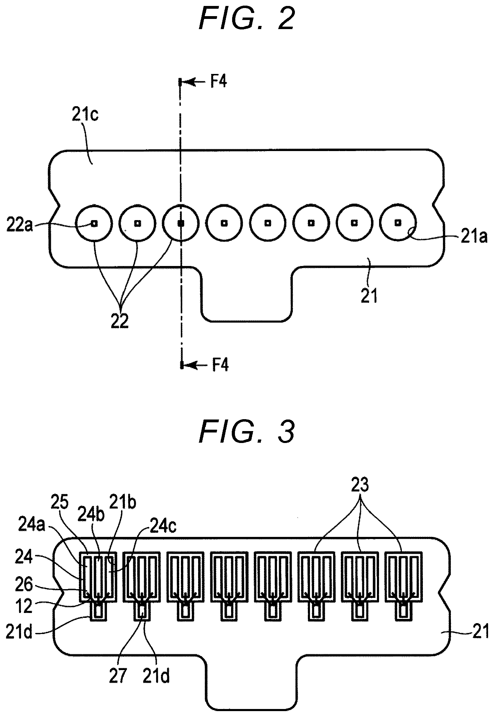

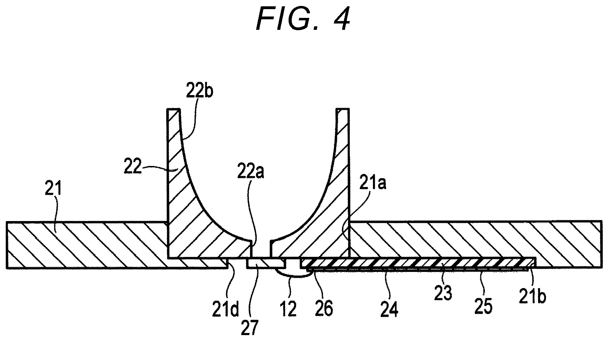

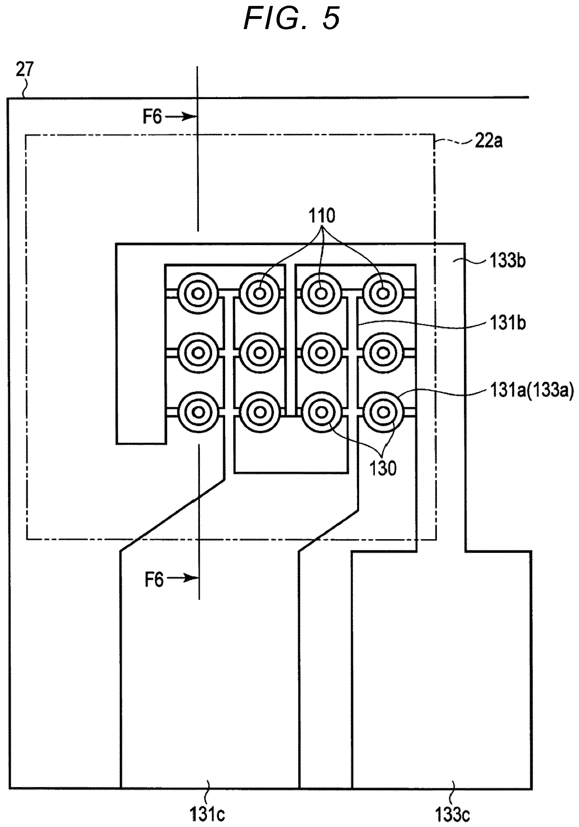

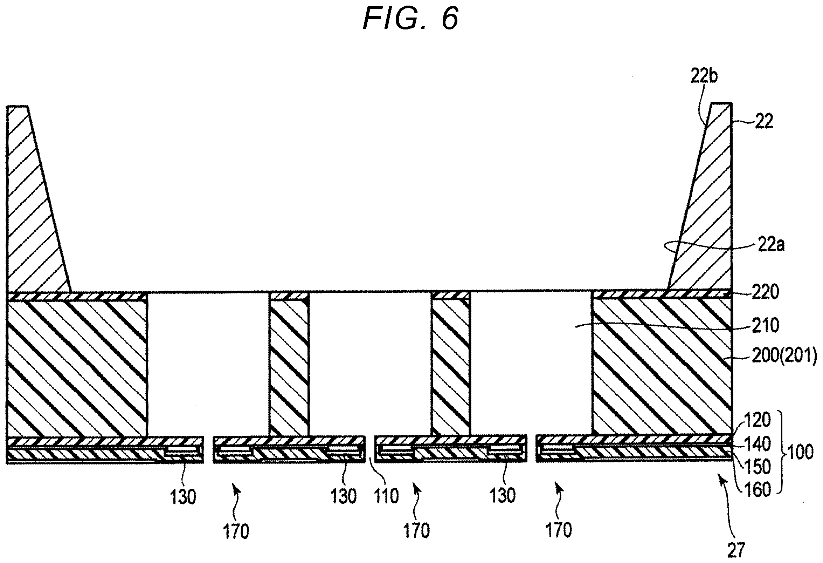

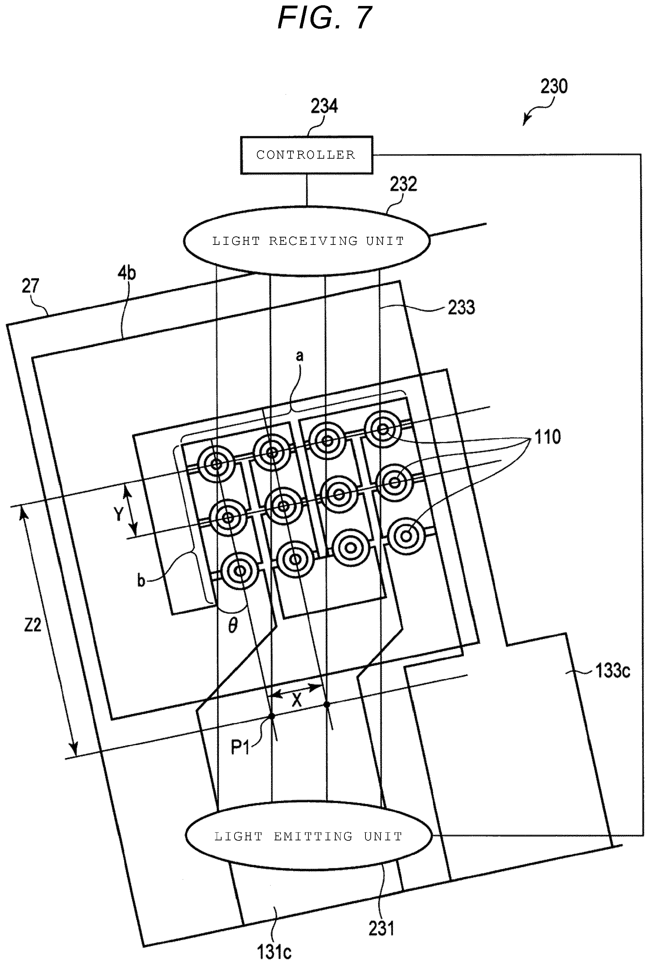

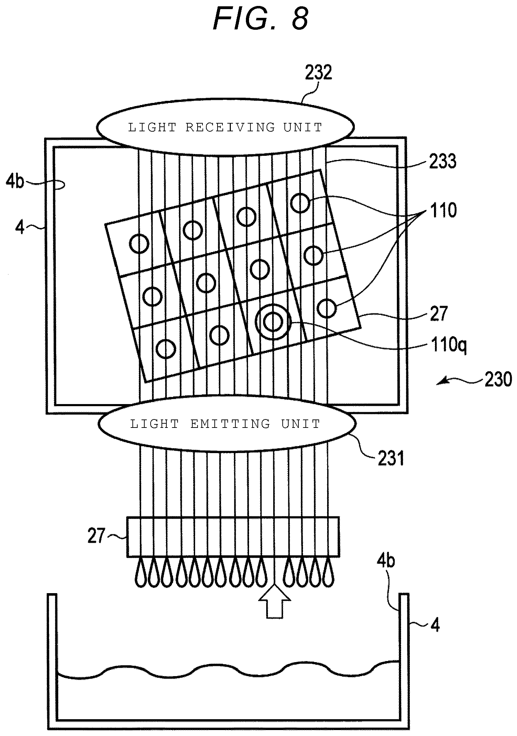

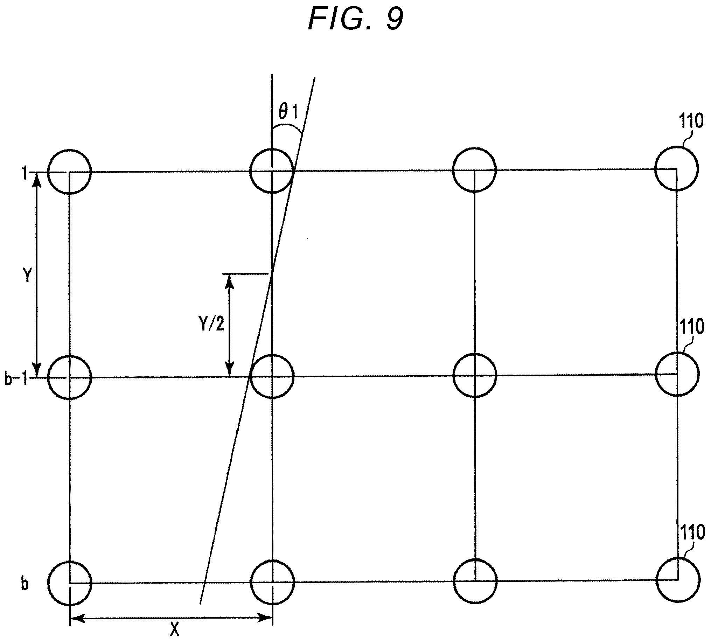

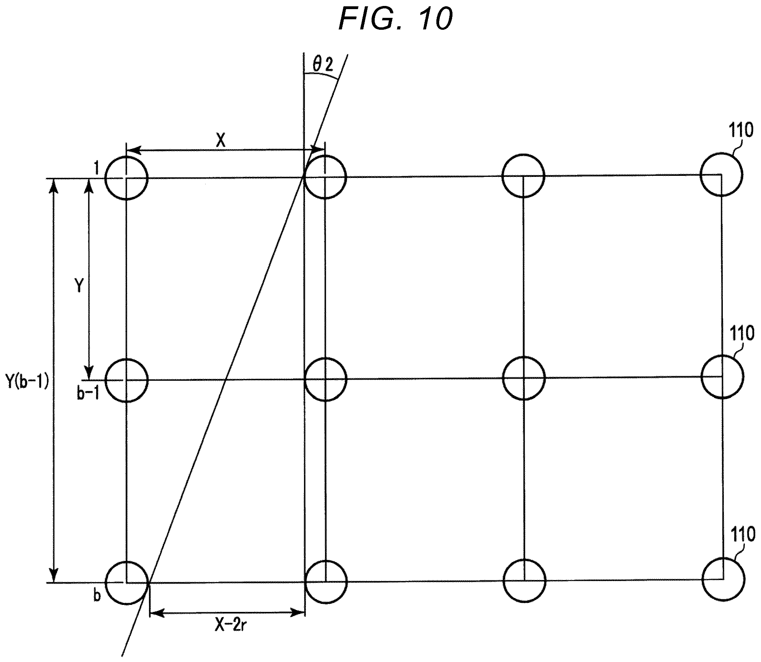

An example of a droplet dispensing apparatus 1 according to a first embodiment will be described with reference to FIG. 1 to FIG. 10. FIG. 1 is a perspective view of the droplet dispensing apparatus 1 according to the first embodiment. FIG. 2 is a plan view of an upper surface of a droplet ejecting apparatus 2 mounted on the droplet dispensing apparatus 1. FIG. 3 is a plan view of a lower surface of the droplet ejecting apparatus 2 from which a droplet is ejected. FIG. 4 is a cross-sectional view along line F4-F4 of FIG. 2. FIG. 5 is a plan view of a droplet ejection array 27 of the droplet ejecting apparatus 2. FIG. 6 is a cross-sectional view along line F6-F6 of FIG. 5. FIG. 7 is a schematic diagram of a droplet detection unit 230 of the droplet dispensing apparatus 1. FIG. 8 is a diagram of a droplet detection unit 230 of the droplet dispensing apparatus 1. FIG. 9 depicts a minimum angle $\theta 1$ of an inclination angle $\theta$ of an optical path 233 of the droplet detection unit 230. FIG. 10 depicts a maximum angle $\theta 2$ of the inclination angle $\theta$ of the optical path 233 of the droplet detection unit 230.
The droplet dispensing apparatus 1 includes a main body 1A having a rectangular flat baseplate 3 and a mounting module 5. In the example embodiment explained herein, a microplate 4, which may also be referred to as a receiving unit, a multiwell plate, or a microwell plate in some context, has 96 wells into which a solution can be dispensed. Microplates having 96 wells are commonly used in a biochemical research and clinical examination. The microplate 4 is not limited to having 96 wells, and may have other number of wells, such as 384 wells, 1536 wells, 3456 wells, 6144 wells, or the like.
The microplate 4 is disposed at the center position of the baseplate 3, and can be secured to and detached from a plate attachment portion 3a of the baseplate 3. There are a pair of X-direction guide rails 6a and 6b extending in a X-direction on both sides of the microplate 4, on the baseplate 3. The ends of each of the X-direction guide rails 6a and 6b are fixed to fixing bases 7a and 7b protruding on the microplate 4.
A Y-direction guide rail 8 extending in a Y-direction is installed between the X-direction guide rails 6a and 6b. Both ends of the Y-direction guide rail 8 are fixed to X-direction moving bases 9 which can slide in the X-direction along the X-direction guide rails 6a and 6b, respectively.
In the Y-direction guide rail 8, a Y-direction moving base 10 is provided, in which the mounting module 5 is movable in the Y-direction along the Y-direction guide rail 8. The mounting module 5 is mounted on the Y-direction moving base 10. The droplet ejecting apparatus 2, which is a droplet ejecting unit, is fixed to the mounting module 5. Thus, the droplet ejecting apparatus 2 can move to any position in X and Y directions which are orthogonal, by the combination of a movement of the Y-direction moving base 10 along the Y-direction guide rail 8 in the Y-direction and a movement of the X-direction moving bases 9 along the X-direction guide rails 6a and 6b in the X-direction. In addition, the droplet ejecting apparatus 2 may be detached from and attached to the mounting module 5.

The droplet ejecting apparatus 2 according to the first embodiment has a flat base plate 21. As shown in FIG. 2, on a surface of the base plate 21 eight solution holding containers 22 are aligned in a row in the Y-direction. In some embodiments, the base plate 21 may have more or less than eight solution holding containers 22. The solution holding container 22 is a bottomed cylindrical container and an upper side is open as shown in FIG. 4. On the bottom surface of the base plate 21, a cylindrical recessed portion 21a is formed at a position corresponding to each solution holding container 22. The bottom of the solution holding container 22 is adhesively fixed to the recessed portion 21a. Further, at the bottom of the solution holding container 22, a solution outlet opening 22a (referred simply to as an opening hereinafter), through which solution is ejected, is formed at the center position. An opening area of a top opening 22b of the solution holding container 22 is larger than an opening area of the opening 22a.

As shown in FIG. 3, an electrical mounting board 23 is provided at each of the solution holding containers 22 on the back side of the base plate 21. The electrical mounting board 23 is a rectangular flat plate. As shown in FIG. 4, a rectangular recessed portion 21b for mounting the electrical mounting board 23 and a droplet ejection opening 21d communicating with the recessed portion 21b are formed on the back side of the base plate 21. Circumference of the recessed portion 21b extends from the solution holding container 22 towards an end of the base plate 21 (an upper end in FIG. 3 and a right end in FIG. 3). As shown in FIG. 4, a portion of the recessed portion 21b overlaps with the solution holding container 22. The electrical mounting board 23 is adhesively fixed to the recessed portion 21b.

On the electrical mounting board 23, an electrical mounting board wiring 24 is patterned on the side opposite to the recessed portion 21b. Three wiring patterns 24a, 24b, and 24c respectively connected to a terminal portion 131c of a lower electrode 131 and two terminal portions 133c of an upper electrode 133 are formed in the electrical mounting board wiring 24.

An input signal control terminal 25 for receiving an external control signal is formed at one end of the electrical mounting board wiring 24. An electrode terminal connector 26 is provided at the other end of the electrical mounting board wiring 24. The electrode terminal connector 26 electrically connects the lower electrode terminal portion 131c and the upper electrode terminal portion 133c formed in the droplet ejection array 27 shown in FIG. 5.

In the base plate 21, the droplet ejection opening 21d is provided. As shown in FIG. 3, the droplet ejection opening 21d is a rectangular through-hole, and is formed at a position overlapping the recessed portion 21a on the back side of the base plate 21.

The droplet ejection array 27 shown in FIG. 5 is adhesively fixed to the lower surface of the solution holding container 22 so as to cover the opening 22a of the solution holding container 22. The droplet ejection array 27 is disposed at a position corresponding to the droplet ejection opening 21d of the base plate 21.

As shown in FIG. 6, the droplet ejection array 27 is formed by stacking a nozzle plate 100 and a pressure chamber structure 200. The nozzle plate 100 includes a nozzle 110 for discharging liquid, a diaphragm 120, a driving element 130, a protective film 150, and a liquid repelling film 160. An actuator 170 is formed with the diaphragm 120 and the driving element 130. In the example embodiment described herein, the actuator 170 may be a piezoelectric element made of a lead-free material containing no lead component, or a piezoelectric element made of lead-containing material.

As shown in FIG. 5, the droplet ejection array 27 has a nozzle group in which a plurality of nozzles is arranged in a X-Y plane that is parallel to the X-direction and the Y-direction. In the example described herein, three nozzles 110 are disposed in a vertical direction (also referred to as a first direction), four nozzles 110 are disposed in a horizontal direction (also referred as a second direction), and one set of twelve nozzles 110 arranged in $3 \times 4$ rows and columns is defined as a nozzle group. That is, in the example embodiment described herein, as shown in FIG. 5, a plurality of nozzles 110 is disposed in each of the first direction and the second direction. The terminal portion 131c of the lower electrode 131 is spaced from the nozzle group in the first direction, and the terminal portion 131c and other terminal portions 131c for other nozzle groups are aligned in the second direction.

Furthermore, in the droplet ejection array 27 according the present example embodiment, twelve nozzles in one nozzle group are disposed at a position corresponding to one opening 22a of one of the eight solution holding containers 22. The twelve nozzles 110 of one nozzle group are disposed only within one well opening 4b of the microplate 4.

The diaphragm 120 is formed integrally with the pressure chamber structure 200, for example. The driving element 130 is formed for each nozzle 110. The driving element 130 has an annular shape surrounding the nozzle 110. The shape of the driving element 130 is not limited, and may be, for example, a C-shape in which a part of a circular ring is cut out.

The diaphragm 120 deforms in the thickness direction by the action of the planar driving element 130. The droplet ejecting apparatus 2 discharges the solution supplied to the nozzle 110 due to the pressure change occurring in the pressure chamber 210 of the pressure chamber structure 200 caused by the deformation of the diaphragm 120.

The main body 1A of the droplet dispensing apparatus 1 includes a droplet detection unit 230 shown in FIG. 7. The droplet detection unit 230 includes a light emitting unit 231, a light receiving unit (a light receiving sensor) 232, and a controller 234. The light emitting unit 231 includes, for example, a light source having a plurality of LED elements in a row. Further, the light receiving unit 232 includes, for example, a CCD camera. The controller 234 includes, for example, a microprocessor, and is connected to the light emitting unit 231 and the light receiving unit 232. The light emitting unit 231 and the light receiving unit 232 may be formed integrally in the droplet ejecting apparatus 2, or may be provided in the mounting module 5.

The light emitting unit 231 and the light receiving unit 232 are disposed at either sides of a nozzle group along a direction in which droplets are discharged from the nozzle group. Along an optical path 233 between the light emitting unit 231 and the light receiving unit 232, substantially horizontally-polarized light is emitted from the light emitting unit 231 to the light receiving unit 232. The droplet detection unit 230 is driven by the controller 234. When droplets shield the optical path 233 between the light emitting unit 231 and the light receiving unit 232, light intensity received by the light receiving unit 232 decreases. The controller 234 receives an output corresponding to the light intensity detected by the light receiving unit 232. When the detected light intensity is less than a specified amount, the controller 234 detects droplets are being discharged from the nozzle 110.

In the droplet ejection array 27, one nozzle group is disposed at the position corresponding to each of the eight solution holding containers 22. Therefore, the droplet detection unit 230 is provided in each of the eight nozzle groups.

In the present example embodiment described herein, as shown in FIG. 8, the optical path 233 between the light emitting unit 231 and the light receiving unit 232 is disposed obliquely with respect to the first direction and the second direction, along the column and the rows of one nozzle group of the droplet ejection array 27. The inclination angle θ of the optical path 233 with respect to the columns of the nozzles 110 in the first direction (also referred to as a nozzle installation direction) is set based on the following constraint condition.

As shown in FIG. 8, a nozzle group is disposed in a matrix in which the number of columns in the first direction is a and the number of rows in the second direction is b. The distance between adjacent columns is X, the distance between the adjacent rows is Y, and the inclination angle of the optical path 233 with respect to the columns in the first direction is θ, which satisfies 0 < θ < 90 (an acute angle).

Further, a distance between a center point of the nozzle 110 in the first column (the leftmost column) and the first row (topmost column) and an intersection point p1 between a straight line through the center points of the nozzles 110 in the first column and an optical axis of the optical path 233 passing through the nozzle 110 in the second column (the second leftmost column) and the first row is defined as Z2.

The inclination angle θ satisfies the following equations (1), (2), and (3).

$$Z2 > Y \cdot (b\text{-}1) \qquad (1)$$

$$\tan\theta = X/Z2 \qquad (2)$$

$$0 < \tan\theta < X/Y \cdot (b\text{-}1) \qquad (3)$$

FIG. 9 is a schematic diagram of a minimum inclination angle θ1 of an optical path 233 of the droplet detection unit 230. The minimum inclination angle θ1 required for detection of a droplet is determined by a size of a droplet ejected from the nozzle 110. When a diameter of the nozzle 110 is r, which is about 10 to 40 μm, the minimum inclination angle θ1 satisfies the following condition.

[Expression 1]
$$tan\theta_1 = \frac{r}{Y/2}$$

FIG. 10 is explanatory schematic diagram of a maximum angle θ2 of the optical path 233 of the droplet detection unit 230. The maximum angle θ2 required for detection of droplets from each column of nozzles is determined by the size of a droplet and the distance between adjacent columns of nozzles. The maximum θ2 satisfies the following condition.

[Expression 2]
$$tan\theta_2 = \frac{X-2r}{Y(b-1)}$$

In the droplet dispensing apparatus 1 according to the first embodiment, the droplet ejection array 27 of the droplet ejecting apparatus 2 is mounted on the mounting module 5. When the droplet ejecting apparatus 2 is in use, a predetermined amount of solution is supplied to the solution holding container 22 from the top opening 22b of the solution holding container 22 by a pipette or the like (not shown). The solution is held on the inner surface of the solution holding container 22. The opening 22a at the bottom of the solution holding container 22 is fluidly connected to the droplet ejection array 27. The solution held in the solution holding container 22 flows into each pressure chamber 210 of the droplet ejection array 27 through the opening 22a.

A voltage control signal that is input to the input signal control terminal 25 is transmitted from the electrode terminal connector 26 to the terminal portion 131c of the lower electrode 131 and the terminal portion 133c of the upper electrode 133. In response to the voltage control signal applied to the driving element 130, the diaphragm 120 is deformed to change the volume of the pressure chamber 210, and thus the solution is discharged as solution droplets from the nozzle 110 of the droplet ejection array 27. In the example embodiment described herein, solution droplets are simultaneously dropped from the twelve nozzles 110 to one well opening 4b of the microplate 4. A predetermined amount of liquid is dropped to each well opening 4b of the microplate 4 from the nozzle 110.

An amount of liquid that is dropped is controlled by a number of repetitions of one-droplet dropping from each nozzle 110, and thus it is possible to control dropping of a liquid to each well opening 4b in the order of picoliter (pL) to microliter ($\mu$L).

In the present embodiment, the droplet detection unit 230 is driven at the same time during an operation of dropping solution droplets from the nozzle 110 of the droplet ejection array 27. Since the light intensity received by the light receiving unit 232 decreases when the droplet shields the optical path 233 between the light emitting unit 231 and the light receiving unit 232, the droplet detection unit 230 detects droplets discharged from the nozzle 110.

The principle of an operation of detecting droplets by the droplet detection unit 230 of this embodiment will be described with reference to FIG. 8. FIG. 8 shows an example in which a nozzle group having twelve nozzles 110 arranged in $3 \times 4$ rows and columns as a set is used, as an example of the nozzle group of the droplet ejection array 27. Then, in a case where clogging occurs in any one of the twelve nozzles 110 arranged in $3 \times 4$ rows and columns, the nozzle 110 in which clogging occurs is indicated as a nozzle 110q. No droplet drops from the nozzle 110q.

As shown in FIG. 8, in the droplet detection unit 230, the optical path 233 between the light emitting unit 231 and the light receiving unit 232 is disposed obliquely with respect to the nozzle installation direction of one nozzle group of the droplet ejection array 27. Therefore, it is possible to simultaneously detect all droplets dropped from twelve nozzles 110 arranged in $3 \times 4$ rows and columns of one nozzle group by the light receiving unit 232. For example, when droplets are dropped from the nozzles 110 other than the nozzle 110q, a decrease in the light intensity is detected by the light receiving unit 232. However, since the droplet is not dropped from the nozzle 110q, there is no decrease in the light intensity received by the light receiving unit 232 at the position corresponding to the nozzle 110q.

Based on the detected light intensity by the light receiving unit 232, the nozzle 110q which is clogged can be detected by a processor (not shown). Further, since no nozzles are aligned in a direction parallel to the optical path 233 between the light emitting unit 231 and the light receiving unit 232, there is no possibility droplets that have been ejected from nozzles block a path for a droplet of the clogged nozzle would have been dropped.

In the droplet dispensing apparatus 1 according to the first embodiment, a droplet detection unit 230 includes a processor (not shown) and is driven during an operation of dropping solution droplets from the nozzle 110 of the droplet ejection array 27. As shown in FIG. 8, in the droplet detection unit 230, the optical path 233 between the light emitting unit 231 and the light receiving unit 232 is disposed obliquely with respect to the nozzle installation direction of the columns of the nozzles 110 in the first direction of a nozzle group of the droplet ejection array 27. Therefore, since all droplets dropped from twelve nozzles 110 arranged in one nozzle group can be simultaneously detected by the light receiving unit 232, it is possible to accurately detect a nozzle 110 which does not discharge, in reference to the detected light intensity by the light receiving unit 232. As a result, when the liquid is simultaneously dropped from twelve nozzles 110 in one nozzle group into one well opening 4b of the microplate 4, it is possible to detect a discharge failure, such as a clogging, in a nozzle 110 in the nozzle group. When a discharge failure is detected, and thus a predetermined amount of liquid cannot be dropped from the droplet ejection array 27 into the well opening 4b of the microplate 4, the controller 234 can quickly stop the dropping of the solution from the nozzle 110. Thus, it is possible to stop the dropping of liquid at an early stage when a discharge failure occurs, which contributes to reduction of waste in a dose-response or the like, and early error detection in an evaluation result of drug performance. As a result, it is possible to provide a droplet dispensing apparatus which can provide more accurate evaluation results of drug performance.

A piezoelectric element may be made of a lead-free material that has lower piezoelectric characteristics than a piezoelectric element including a lead component, for example, PZT (Pb(Zr, Ti)O3: lead titanate zirconate). Therefore, in the case of the piezoelectric element made of a lead-free material, the amount of displacement of the diaphragm 120 during driving is smaller than that of the piezoelectric element made of PZT, so that the amount of liquid per drop is small.

In the example embodiment described herein, a plurality of nozzles 110 (12 nozzles arranged in $3 \times 4$ rows and columns) is disposed in one nozzle group for one well opening 4b. Thus, even with a lead-free piezoelectric element having low piezoelectric characteristics, it is possible to speed-up the dropping of the required amount of liquid. Therefore, it is possible to complete the dropping of the necessary amount of the liquid in a short time to all the well openings 4b of the microplate 4.

Second embodiment

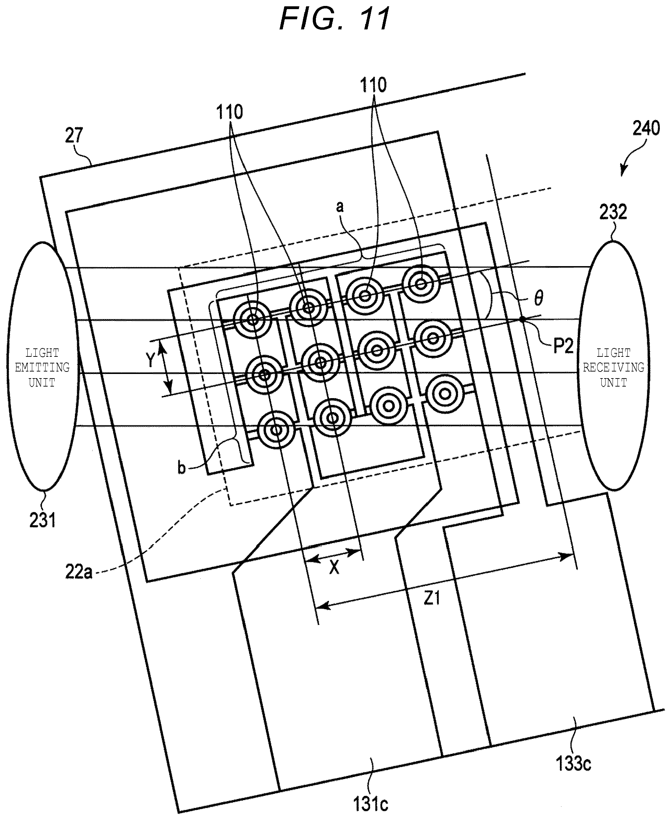

FIG. 11 is a perspective view of a droplet detection unit of a droplet dispensing apparatus according to a second embodiment. In this example embodiment, the droplet detection unit 230 of the droplet dispensing apparatus 1 according to the first embodiment is modified as follows. The same reference numerals are used for the components that are substantially the same as those of the first embodiment, and the detailed description of repeated components may be omitted.

In the first embodiment, the optical path 233 between the light emitting unit 231 and the light receiving unit 232 is disposed obliquely with respect to the nozzle installation direction along the columns of the nozzles 110 in the first direction. In the second embodiment, a droplet detection unit 240 is provided in which the optical path 233 between the light emitting unit 231 and the light receiving unit 232 is disposed obliquely with respect to the nozzle installation direction of the rows of the nozzles 110 in the second direction.

Similar to the droplet detection unit 230 according to the first embodiment, since the intensity of light received by the light receiving unit 232 decreases when the droplet shields the optical path 233 between the light emitting unit 231 and the light receiving unit 232, the droplet detection unit 240 according to the second embodiment can also detect droplets discharged from the nozzle 110.

Third embodiment

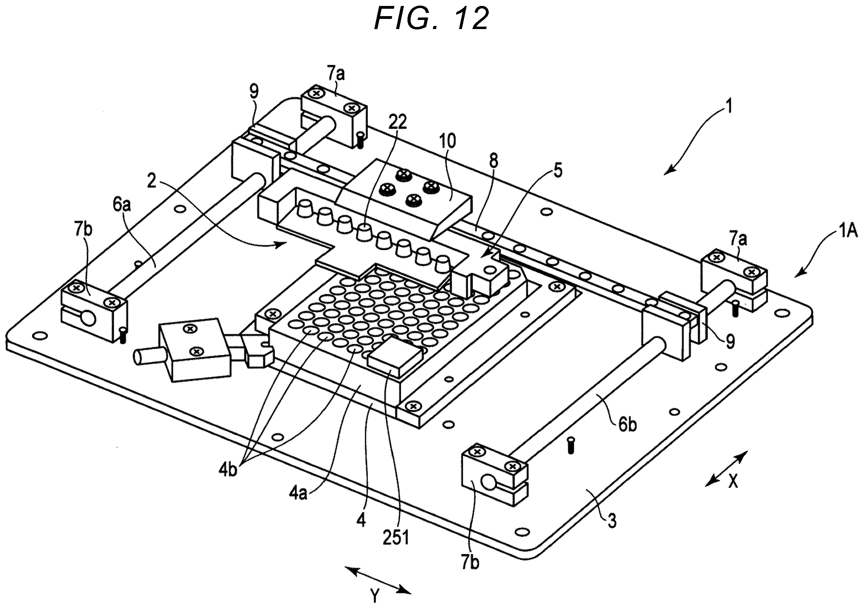

FIG. 12 is a perspective view of a droplet detection unit of a droplet dispensing apparatus according to a third embodiment. In this example embodiment, the droplet dispensing apparatus 1 according to the first embodiment is modified as follows. The same reference numerals are used for the components that are substantially the same as those of the first embodiment, and the detailed description of repeated components may be omitted.

In the third embodiment, a second droplet ejecting unit 251 in addition to the droplet ejection array 27 is provided. The second droplet ejecting unit 251 has a support mechanism which supports the droplet ejecting unit 2 so as to be movable to an arbitrary position in the X-Y direction separately from the droplet ejecting apparatus 2.

The second droplet ejecting unit 251 includes, for example, a water tank (not shown). The second droplet ejecting unit 251 may further include a tank that contains the same liquid as that of the droplet ejection array 27.

In the third embodiment, after a predetermined amount of liquid has been dropped from the droplet ejection array 27 into each well opening 4b of the microplate 4, when a preset set time elapses, a solution (water) is additionally discharged from the second droplet ejecting unit 251 into each well opening 4b of the microplate 4. This makes it possible to prevent the drying of the cells contained in each well opening 4b of the microplate 4, in a case where the liquid contained in each well opening 4b of the microplate 4 may dry when in contact with air.

For example, in a high-density microplate, there is a possibility that cells dry due to liquid evaporation during dropping, due to an increase in dropping time and a decrease in liquid amount due to an increase in the number of wells. In such a case, it is possible to effectively prevent the drying of the cells contained in each well opening 4b of the microplate 4, by dispensing an additional solution by the second droplet ejecting unit 251. This enables high-efficiency experiment using the high-density microplate.

Furthermore, the support mechanism of the second droplet ejecting unit 251 may be able to perform a parallel process of dispensing a droplet in parallel or perpendicular to the droplet ejection array 27.

Fourth embodiment

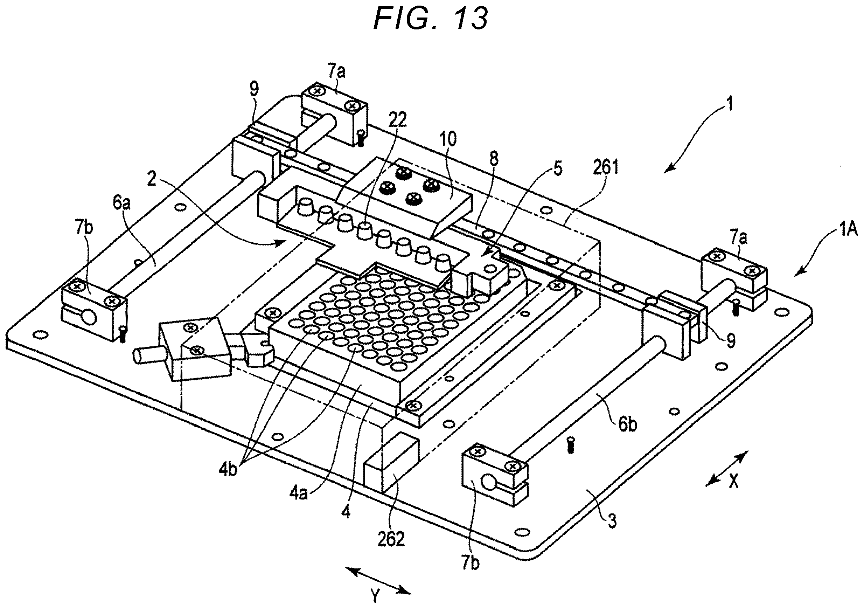

FIG. 13 is a perspective view of a droplet detection unit of a droplet dispensing apparatus according to a fourth embodiment. In this example embodiment, the droplet dispensing apparatus 1 according to the first embodiment is modified as follows. The same reference numerals are used for the components that are substantially the same as those of the first embodiment, and the detailed description of repeated components may be omitted.

In the fourth embodiment, a sealed box component 261 that encloses the microplate 4 and a spraying device 262 spraying a humidifying solution inside the sealed box component 261 are provided on the baseplate 3. The sealed box component 261 includes, for example, a frame portion having a highly rigid frame structure and a cover made of an elastic material for closing a space between the frame portions of each frame. The the sealed box component 261 can be hermetically sealed by the frame portion and the cover.

The spraying device 262 includes, for example, a water tank (not shown). The spraying device 262 may further include a tank that contains the same liquid as that of the liquid ejection array 27. The spraying device 262 is provided in the sealed box component 261, and sprays the liquid droplets to the inner space of the sealed box component 261 for drying prevention.

In the fourth embodiment, after a predetermined amount of liquid has been dropped from the droplet ejection array 27 into each well opening 4b of the microplate 4, when a preset set time elapses, droplets for preventing drying are sprayed from the spraying device 262 into the inner space of the sealed box component 261. The spraying device 262 may be configured such that droplets for drying prevention are sprayed simultaneously with the start of the liquid dropping operation from the droplet ejection array 27.

This makes it possible to prevent the drying of the cells contained in each well opening 4b of the microplate 4, in a case where the liquid contained in each well opening 4b of the microplate 4 dries when in contact with air.

Fifth embodiment

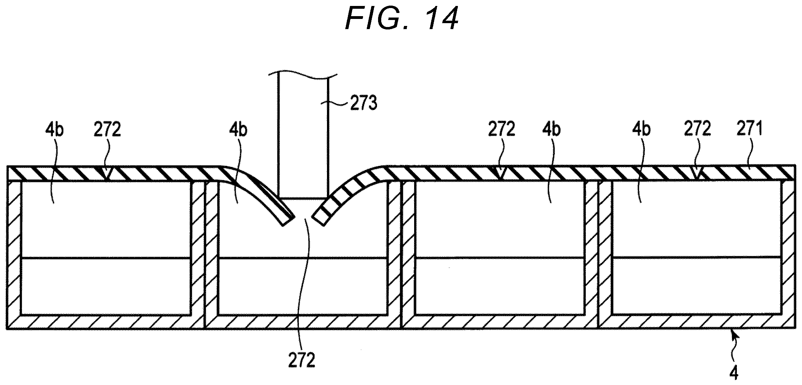

FIG. 14 is a longitudinal cross-sectional view of a microplate 4 according to a fifth embodiment. In the microplate 4 in the present modification example embodiment, a lid 271 made of an elastic material such as rubber is provided on the periphery of the opening of the well opening 4b. In the lid 271, a notch 272 such as a slit is formed at the center position of the opening of the well opening 4b.

A needle-like injection member 273 is provided in the droplet ejection array 27. At the tip end of the injection member 273, there is provided an actuator capable of injecting droplets of a pL order.

In the microplate 4, the opening of the well opening 4b is blocked by the lid 271 in the standby state (when not in use). In this state, the notch 272 of the lid 271 is closed.

At the time of an operation of dropping liquid from the droplet ejection array 27 of the droplet ejecting apparatus 2, as shown in FIG. 14, the tip end of the injection member 273 is press-fitted into the notch 272 of the lid 271. Thus, the tip end of the injection member 273 pries the notch 272 of the lid 271 open. At this time, the lid 271 elastically deforms to a state in which the peripheral portions on both sides of the notch 272 are pushed into the inside of the well opening 4b. Therefore, when the tip end of the injection member 273 is inserted to the inside of the well opening 4b, droplets are discharged from the tip end of the injection member 273.

When a specified number of droplets are discharged from the injection member 273, the injection member 273 is withdrawn to the outside of the microplate 4. At this time, the lid 271 elastically returns to a state in which the peripheral portions on both sides of the notch 272 are closed. Therefore, the well opening 4b of the microplate 4 is closed by the lid 271. As a result, the inner space of the well opening 4b of the microplate 4 is maintained in an airtight state by the lid 271, so that evaporation of the droplet injected into the internal space of the well opening 4b of the microplate 4 is prevented.

As a result, it is possible to prevent the liquid contained in each well opening 4b of the microplate 4 from touching the outside air and drying, and to prevent the drying of the cells contained in each well opening 4b of the microplate 4.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the inventions. The accompanying claims and their equivalents are intended to cover such forms or modifications as would fall within the scope of the inventions.

**Claims**

1. A droplet dispensing apparatus, comprising:

   a droplet ejection array having a plurality of nozzles from which droplets can be ejected into a well opening of a microplate plate on a baseplate, the plurality of nozzles being arranged in columns in a first direction and rows in a second direction that intersects the first direction;
   a light emitting unit configured to emit light having polarization perpendicular to a third direction oblique with respect to the first direction and the second direction; and
   a light receiving unit configured to receive light from the light emitting unit, the light receiving unit being on an opposite side of the droplet ejection array from the light emitting unit.

2. The droplet dispensing apparatus according to claim 1, wherein an inclination angle of the third direction with respect to the first direction is an acute angle.

3. The droplet dispensing apparatus according to claim 2, wherein when the number of columns is a, the number of rows is b, a distance between adjacent columns is X, a distance between adjacent rows is Y, and the inclination angle is θ, a distance between a first nozzle, disposed in a first column and a first row, and an intersection point between a line through the first nozzle along the first direction and a line through a second nozzle, disposed in a second column and the first row, along the third direction is Z2, the second column being adjacent to the first column, the following relationships:

$$Z2 > Y \cdot (b\text{-}1),$$

and

$$0 < \tan\theta < X/Y \cdot (b\text{-}1)$$

**4.** The droplet dispensing apparatus according to claim 1, wherein an inclination angle of the third direction with respect to the second direction is an acute angle.

**5.** The droplet dispensing apparatus according to any one of claims 1 to 4, further comprising:
an additional droplet ejection unit configured to dispense liquid towards the microplate on the baseplate.

**6.** The droplet dispensing apparatus according to any one of claims 1 to 5, further comprising:

a sealed box enclosing the droplet ejection array and at least a portion of the baseplate; and
a spraying device and configured to spray a liquid into the sealed box.

**7.** The droplet dispensing apparatus according to any one of claims 1 to 6, further comprising:
a needle-like ejection member on the droplet ejection array configured to engage and open a lid on a periphery of a well opening of the microplate.

**8.** The droplet dispensing apparatus according to any one of claims 1 to 7, further comprising:

a board having a first surface and a second surface, wherein the droplet ejection array is on the first surface of the board and the droplets can be ejected via the second surface of the board;
a plurality of pressure chambers on the second surface of the board, each pressure chamber in the plurality being fluidly connected to a respective nozzle in the plurality of nozzles;
a plurality of actuators that changes a pressure in a respective pressure chamber and causes liquid to be discharged from the respective nozzle; and
a plurality of solution holding containers on the second surface of the board, each having a solution receiving port for receiving liquid and a solution outlet for supplying liquid to a respective pressure chamber.

**9.** The droplet dispensing apparatus according to claim 8, wherein the light emitting unit and the light receiving unit are formed integrally with the droplet ejecting array.

**10.** The droplet dispensing apparatus according to any one of claims 1 to 9, further comprising a controller configured to receive an output corresponding to light intensity detected by the light receiving unit.

**11.** The droplet dispensing apparatus according to claim 10, wherein the controller is further configured to detect droplets being discharged from a nozzle when the detected light intensity is less than a specified amount.

**12.** An analytic system comprising the droplet dispensing apparatus according to any one of claims 1 to 11.

FIG. 1

# FIG. 2

# FIG. 3

## FIG. 4

# FIG. 5

*FIG. 6*

# FIG. 7

FIG. 8

## FIG. 9

EP 3 378 560 A1

FIG. 10

EP 3 378 560 A1

# FIG. 11

*FIG. 12*

FIG. 13

FIG. 14

**EP 3 378 560 A1**

Europäisches
Patentamt

European
Patent Office

Office européen
des brevets

## EUROPEAN SEARCH REPORT

Application Number

EP 18 15 9392

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2001/043245 A1 (ENDO HIRONORI [JP] ET AL) 22 November 2001 (2001-11-22) | 1-5,9-12 | INV. B01L3/02 |
| Y | * paragraph [0114] - paragraph [0138] * <br> * paragraph [0138] * <br> * paragraph [0172] - paragraph [0173]; figure 16 * | 6-8 | G01N35/10 <br> B41J2/14 <br> B41J2/165 |
| Y | US 2002/001546 A1 (HUNTER IAN [US] ET AL) 3 January 2002 (2002-01-03) <br> * paragraph [0132] * <br> * paragraph [0090] * | 6,7 | |
| Y | JP 2017 013042 A (TOSHIBA TEC KK) 19 January 2017 (2017-01-19) <br> * paragraph [0039] - paragraph [0042] * | 8 | |

TECHNICAL FIELDS
SEARCHED      (IPC)

B01L
G01N
B41J

The present search report has been drawn up for all claims

| Place of search | Date of completion of the search | Examiner |
|---|---|---|
| The Hague | 23 May 2018 | Ueberfeld, Jörn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 15 9392

This annex lists the patent family members relating to the patent documents cited in the above-mentioned European search report.
The members are as contained in the European Patent Office EDP file on
The European Patent Office is in no way liable for these particulars which are merely given for the purpose of information.

23-05-2018

| Patent document cited in search report | | Publication date | Patent family member(s) | | Publication date |
|---|---|---|---|---|---|
| US 2001043245 | A1 | 22-11-2001 | AT | 324983 T | 15-06-2006 |
| | | | DE | 60119191 T2 | 12-04-2007 |
| | | | EP | 1127694 A1 | 29-08-2001 |
| | | | US | 2001043245 A1 | 22-11-2001 |
| US 2002001546 | A1 | 03-01-2002 | CA | 2446157 A1 | 14-11-2002 |
| | | | CA | 2665706 A1 | 14-11-2002 |
| | | | EP | 1385628 A2 | 04-02-2004 |
| | | | JP | 4607202 B2 | 05-01-2011 |
| | | | JP | 4741042 B2 | 03-08-2011 |
| | | | JP | 5103536 B2 | 19-12-2012 |
| | | | JP | 2005507492 A | 17-03-2005 |
| | | | JP | 2008268202 A | 06-11-2008 |
| | | | JP | 2008292478 A | 04-12-2008 |
| | | | JP | 2009096992 A | 07-05-2009 |
| | | | JP | 2011095271 A | 12-05-2011 |
| | | | US | 2002001546 A1 | 03-01-2002 |
| | | | US | 2004191924 A1 | 30-09-2004 |
| | | | US | 2005079105 A1 | 14-04-2005 |
| | | | US | 2009258797 A1 | 15-10-2009 |
| | | | US | 2011319300 A1 | 29-12-2011 |
| | | | WO | 02089982 A2 | 14-11-2002 |
| JP 2017013042 | A | 19-01-2017 | JP | 2017013042 A | 19-01-2017 |
| | | | US | 2017165969 A1 | 15-06-2017 |
| | | | US | 2018065361 A1 | 08-03-2018 |

EPO FORM P0459

For more details about this annex : see Official Journal of the European Patent Office, No. 12/82